# EUROPÄISCHE PATENTSCHRIFT

(11) **EP 2 023 856 B1**
(45) Veröffentlichungstag und Bekanntmachung des Hinweises auf die Patenterteilung: **13.08.2014**
(21) Anmeldenummer: 07729450.2
(22) Anmeldetag: 23.05.2007
(51) Int. Cl.: A61F 2/14

(54) **HORNHAUTIMPLANTAT ZUR KORREKTUR VON FEHLSICHTIGKEITEN DES MENSCHLICHEN AUGES**
CORNEAL IMPLANT FOR CORRECTION OF IMPAIRED VISION IN THE HUMAN EYE
IMPLANT CORNÉEN DE CORRECTION DES DÉFAUTS DE LA VISION DE L' OEIL HUMAIN

(30) Priorität: 23.05.2006 AT 8852006
(43) Veröffentlichungstag der Anmeldung: 18.02.2009
(73) Patentinhaber: Daxer, Albert, A-4020 Linz (AT)
(72) Erfinder: Daxer, Albert, A-4020 Linz (AT)
(74) Vertreter: KLIMENT & HENHAPEL
(86) Internationale Anmeldenummer: PCT/EP2007/055015
(87) Internationale Veröffentlichungsnummer: WO 2007/135173

(56) Entgegenhaltungen:
- WO-A-03/020176
- WO-A-03/020190
- US-A1- 2004 143 324
- US-A1- 2006 116 759

## Beschreibung

### GEBIET DER ERFINDUNG

Die vorliegende Erfindung bezieht sich auf ein Hornhautimplantat zur Einbringung in das optische Zentrum der Hornhaut des menschlichen Auges zur Behebung von Fehlsichtigkeiten, insbesondere von Alterssichtigkeiten bei sonst normalsichtigen (emotropen). Augen aber auch von Altersichtigkeiten in Kombination mit Weitsichtigkeiten oder Kurzsichtigkeiten.

Die Erfindung ist geeignet zur Korrektur der Fehlsichtigkeit des menschlichen Auges, insbesondere zur Korrektur von Alterssichtigkeit, Alterssichtigkeit und Weitsichtigkeit sowie Alterssichtigkeit und Kurzsichtigkeit, sowie Altersichtigkeit und Astigmatismus durch Implantation eines Hornhautimplantates im optischen Zentrum der Hornhaut.

Der optische Apparat des Auges zur Abbildung der Umwelt besteht im Wesentlichen aus der Hornhaut und der hinter der Iris gelegenen Linse. Dieser optische Apparat des Auges besitzt eine Gesamtbrechkraft von ungefähr 60 Dioptrien, wobei die Grenzfläche zwischen Hornhaut und Luft, also die äußere Begrenzung des Auges, den Hauptbeitrag mit etwa 40 Dioptrien liefert. Diese Brechkraft der Hornhaut ist im Wesentlichen indirekt proportional zum Radius der Hornhautvorderfläche (Grenzfläche zwischen Hornhaut und Luft). Durch Veränderung des Hornhautradius lässt sich somit auch die Brechkraft des Auges verändern.

Im Falle von Kurzsichtigkeit ist der Augapfel zu lange und die Brechkraft der Hornhaut reicht nicht aus, um eine Fokussierung der einfallenden Lichtstrahlen auf der Netzhaut zu ermöglichen. Anstattdessen erfolgt die Fokussierung vor der Netzhaut.

Im Falle von Weitsichtigkeit ist der Augapfel zu kurz und die Brechkraft der Hornhaut reicht nicht aus, um eine korrekte Fokussierung der einfallenden Lichtstrahlen auf der Netzhaut zu ermöglichen. Anstattdessen erfolgt die Fokussierung hinter der Netzhaut.

Bei der Alterssichtigkeit handelt es sich um eine Dissoziation der Brechkraft des Auges in der Form, dass für das scharfe Sehen in der Ferne eine andere Dioptrienkorrektur notwendig ist, als für das scharfe Sehen in der Nähe.

Es sind unterschiedliche Möglichkeiten zur Behebung dieser Fehlsichtigkeiten bekannt. Neben den klassischen Methoden, die Fehlsichtigkeit mittels Brillen oder Kontaktlinsen zu beheben, sind auch chirurgische Methoden bekannt, bei welchen Implantate in die Hornhaut eines menschlichen Auges eingesetzt werden, um entweder die Krümmung der Hornhaut zu verändern und dadurch deren Brechkraft entsprechend zu korrigieren oder durch entsprechende optische Eigenschaften des Implantats die optischen Eigenschaften der Hornhaut zu verändern.

So führt etwa eine Vergrößerung des Hornhautradius zu einer Abnahme der Brechkräft, womit man Kurzsichtigkeit korrigieren kann. Um Weitsichtigkeit korrigieren zu können, muss der Hornhautradius verringert, also die Krümmung erhöht werden.

Um Alterssichtigkeit chirurgisch zu korrigieren, ist es notwendig der Brechkraft der Hornhaut eine Bi- bzw. Multifokalität aufzuprägen. Das bedeutet, dass die Brechkraft der Hornhaut so gestaltet ist, dass einfallenden Lichtstrahlen aus unterschiedlichen Entfernungen (Ferne oder Nähe) je nach deren Durchtrittspunkt durch die Hornhaut gleichzeitig auf der Netzhaut bzw. im Bereich des Zentrums der Netzhaut (= Makula, Stelle des schärfsten Sehens) abgebildet werden. Das heißt, es wird gleichzeitig ein Bild oder mehrere Bilder des Fernbereiches und ein Bild oder mehrere Bilder des Nahbereiches in der Makula abgebildet. Das Gehirn wählt dann das gewünschte Bild aus. Damit diese Auswahl stattfinden kann, sollte die Abbildung des Fernbereiches und die Abbildung des Nahbereiches annähernd gleiche Intensität haben. Dies wird z.B. bei Kontaktlinsen und bei Intraokularlinsen nach Staroperationen ausgenützt.

### STAND DER TECHNIK

Die US 2004/0143324 A1 zeigt ein Hornhautimplantat zur kosmetischen Verwendung mit einer Breite von 0,5-12 mm mit kosmetischer Verwendung ist gemeint, dass es keinen medizinischen Zwede, d.h. keine Behebung von Fehlsichtigkeit aufweist. Das Implantat ist in Größe, Form, Gewicht und Position in der Verwendung so dimensioniert, dass die normale Funktion des Weges nicht beeinträchliegt wird.

Aus der WO 93/05731 ist es bekannt, eine optische Linse im optischen Zentrum der Hornhaut zu Implantieren, dessen Abmessungen geringer sind als jene der optischen Zone, die durch den Pupillendurchmesser begrenzt ist.

Das optische Zentrum der Hornhaut ist jener Bereich der Hornhaut, entlang dessen die optische Achse des Auges durch die Hornhaut verläuft. Die optische Achse ist die Abbildungsachse des optischen Systems des Auges. Das optische Zentrum legt der Augenspezialist durch Anwendung von Bestimmungsverfahren fest. Der Augenspezialist kann aus einer Vielzahl an unterschiedlichen Verfahren wählen. Die im folgenden beschriebenen Verfahren zur Festlegung des optischen Zentrums der Hornhaut stellen nur einen Auszug aus den vielen vertretenen Vorgangsweisen dar und erheben keinen Anspruch auf Vollständigkeit. So verwenden viele Systeme, insbesondere Excimerlaser-Systeme mit aktivem Eye-tracking, das Zentrum der Pupille bzw. dessen Projektion auf die Hornhautoberfläche oder um einen im Einzelfall festgelegte Distanz dazu entfernt gelegenen Punkt als optisches Zentrum der Hornhaut. Andere übliche Festlegungen zielen auf die Stelle der stärksten Hornhautkrümmung. Tatsächlich ist besonders bei hoher Kurzsichtigkeit zwischen der optischen Achse und der anatomischen Achse eine Winkelabweichung festzustellen, die anatomischen Achse eine Winkelabweichung festzustellen, die als Winkel Kappa bezeichnet wird. Ein weiteres Verfahren bezieht sich auf die sog. Purkinje Reflexe. Das sind Reflexe an der Hornhautvorderfläche, Hornhautrückfläche, Linsenvorderfläche und Linsenrückfläche, die entstehen, wenn der Patient eine vorzugsweise punktförmige Lichtquelle fixiert. Idealerweise decken sich diese Reflexe. Meist ist dies jedoch nicht der Fall und dann wählt man einen dieser Reflexe als optisches Zentrum aus. Häufig wird auch die Mittelposition aus vier Reflexen, oder die Mitte zwischen dieser Mittelposition und der Pupillenmitte, etc. festgelegt. Letztenendes obliegt es der persönlichen Einschätzung, der individuellen Erfahrung und den Vorlieben des Augenspezialisten, wie er das optische Zentrum der Hornhaut festlegt. Im Allgemeinen unterscheiden sich die verschiedenen Methoden zur Festlegung des optischen Zentrums der Hornhaut in ihrem Resultat nicht wesentlich.

In der WO 93/05731 entstehen durch das Implantieren einer optischen Linse im optischen Zentrum der Hornhaut Zonen unterschiedlicher Brechkraft, nämlich einerseits im Bereich der optischen Linse selbst und andererseits im angrenzenden Hornhautgewebe durch die ohnehin bestehende Brechkraft der Hornhaut. Dadurch kann eine Bifokalität bzw. je nach Verlauf der optischen Fläche der implantierten Linse auch eine Multifokalität erzeugt werden. Die Linsen haben eine Dicke in Richtung der optischen Achse des Auges von kleiner 50µm um keine unerwünschte Auslenkung der Hornhaut und Störung der Brechkraft der Linse zu bewirken. Grundsätzlich besteht jedoch der Nachteil, dass durch die neugeschaffenen Grenzflächen optisch negative Phänomene wie Blendungen und Reflexionen auftreten können, die der Patient dann als störend empfindet. Daher muss die optische Fläche extrem hochwertig ausgeführt werden, was bei derart kleinen Abmessungen sehr schwierig und aufwendig ist. Außerdem ist es bekannt, daß an den Grenzflächen von Hornhautimplantaten biologische Ablagerungen auftreten können, die die Funktion der Implantate als optisches Element erheblich beeinträchtigen können.

Die US 6,589,280 B1 beschreibt eine Methode zur Bildung einer multifokalen Hornhaut durch die Implantation von mindestens 50 mikroskopisch kleinen optischen Linsen außerhalb des optischen Zentrums der Hornhaut. Dabei soll jede Linse über eine definierte Brechkraft verfügen, vorzugsweise 1 bis 3 Dioptrien. Die optischen Linsen weisen eine Dicke von ca. 2-3µm auf und eine Breite gemessen in einer Normalebene zur Dickenrichtung von unter 1mm. Die Linsen sind so klein, dass eine Beeinträchtigung der Krümmung der Hornhaut durch Auslenkung der Hornhautoberfläche nicht gegeben ist. Die Korrektur der Fehlsichtigkeit erfolgt ausschließlich aufgrund der unterschiedlichen Brechkraft der einzelnen Linsen. Die beschriebene Methode ist extrem kompliziert und es gelten betreffend die Funktion in einem lebenden Gewebe wieder die obigen Betrachtungen.

Aus der US 5,722,971 ist es bekannt, ein dünnes scheibenförmiges Implantat mit diffraktiver Optik und einer zentralen Ausnehmung zu implantieren. Der äußere Durchmesser ist mit 3mm bis 9mm angegeben. Darüber hinaus werden neben Ringimplantaten noch Ringersatzimplantate vorgestellt. Dabei wird der Ring durch mehrere Einzelimplantate ersetzt, die entlang eines Kreises konzentrisch um das Hornhautzentrum positioniert werden. Durch die "Nichtbesetzung" einzelner Kreispositionen können neben Kurzsichtigkeiten auch reguläre und irreguläre Astigmatismen korrigiert werden. Angaben zu Abmessungen fehlen, aber aus den dargestellten Anwendungen ergibt sich, dass, um denselben Effekt wie die Ringe erreichen zu können, sie etwa dasselbe Volumen wie diese verdrängen mü-ssen und daher, wie auch in den Ausführungsbeispielen gezeichnet, deutlich größer dimensioniert sein und über Abmessungen in der Größenordnung der Pupillenweite, bzw. der Geometrie. Aus den dargestellten Anwendungen folgt, dass es sich um ein langgestrecktes Ellipsoid handeln muss. Solche Implantate sind für eine Anwendung im Bereich der zentralen Hornhaut ungeeignet.

Das gleiche gilt für die US 2004/0073303 A1. Hier liegt in der bevorzugten Ausführungsvariante sogar ein gekrümmtes, langgestrecktes Implantat (Centroid) vor.

Aus dem Stand der Technik ist es somit bekannt, optische Linsen als Hornhautimplantate im optischen Zentrum der Hornhaut zu implantieren. Diese optischen Hornhautimplantate wirken über deren eigene Brechkraft. Sie besitzen eine optisch wirksame Vorder- und/oder Rückfläche und bestehen zwischen optisch wirksamer Vorder- und/oder Rückfläche aus einem Material mit einem bestimmten Brechungsindex, das zusätzlich zum Flächenverlauf die Brechkraft dieser optischen Hornhautimplantate bestimmt. Allerdings ist es bekannt, dass es bei solchen optischen Hornhautimplantaten an den Grenzflächen zum umgebenden Gewebe, neben rein optischen Grenzflächenphänomenen zu Einlagerungen von biologischem Material kommen kann. Dies führt insbesondere bei Implantaten, die im optischen Zentrum des menschlichen Auges implantiert sind zu einer erheblichen Beeinträchtigung der Sehleistung.

Außerhalb des optischen Zentrums positionierte Implantate sind zwar unempfindlicher gegenüber den erwähnten Einlagerungen, jedoch nicht in der Lage Bifokalität geschweige denn Multifokalität zu erzeugen, um Altersichtigkeit alleine oder in Kombination mit Weitsichtigkeit oder Kurzsichtigkeit korrigieren zu können.

Die US 2004/0143324 A1 zeigt ein Hornhautimplantat zur kosmetischen Verwendung mit einer Dicke im Bereich von 1 bis 250 Mikrometer, wobei die Dicke des Implantates vorzugsweise so gewählt wird, dass es zu keiner signifikanten Gewebsverschiebung bzw. -deformation kommt.

Die WO 03/020190 Aund die WO 03/020176 A betreffen Barrieren zur Korrektur von Alterssichtigkeit. Die Barrieren werden in die Lederhaut eingesetzt, um radiale Einschnitte in der Lederhaut offen zu halten. Die Barrieren haben eine Länge von mehr als 2 mm.

### DARSTELLUNG DER ERFINDUNG

Es ist daher die Aufgabe der vorliegenden Erfindung, ein Hornhauptimplantat vorzuschlagen, welches zum Einsatz im optischen Zentrum des menschlichen Auges geeignet ist und das zur Korrektur von Alterssichtigkeit alleine und in Kombination mit Weitsichtigkeit oder Kurzsichtigkeit eingesetzt werden kann.

Erfindungsgemäß wird diese Aufgabe durch die Merkmale des Anspruchs 1 gelöst. Dabei ist vorgesehen, dass die Wirkdicke des Hornhautimplantats, gemessen in Richtung der optischen Achse des Auges, mehr als 50µm beträgt und die größte Breite, 0,5 gemessen in einer Normalebene zur Dickenrichtung, kleiner als mm ist und das Hornhautimplantat in Bezug auf das menschliche Auge keine Abbildungsfunktion besitzt.

Ein Hornhautimplantat mit den gewählten Dimensionen ist einerseits dazu geeignet, im optischen Zentrum des menschlichen Auges positioniert zu werden, ohne die Sehfähigkeit des menschlichen Auges zu beeinträchtigen und andererseits dazu geeignet, Alterssichtigkeit durch Veränderung der Krümmung der Hornhaut infolge Auslenkung der Hornhautoberfläche in deren optischem Zentrum zu korrigieren. Da ein erfindungsgemäßes Hornhautimplantat in Bezug auf das menschliche Auge keinerlei Abbildungsfunktion aufweist, dh. mit anderen Worten keine optische Wirkung besitzt, ist es einfach in der Herstellung. Die erfindungsgemäßen Dimensionen ermöglichen die Implantation direkt im optischen Zentrum des Auges ohne die Sehfähigkeit des Auges zu verringern. Dadurch kann in weiterer Folge in der Umgebung des Hornhautimplantates infolge der zentralen Volumsaddition ein asphärischer Oberflächenverlauf der Hornhaut erzeugt werden, der eine multifokale Abbildung ermöglicht, um Alterssichtigkeit korrigieren zu können. Weitsichtigkeitskorrekturen sind damit ebenfalls möglich. Die Implantation im optischen Zentrum der Hornhaut bedeutet, dass das Implantat unter Berücksichtigung der endlichen Bestimmungsgenauigkeit des optischen Zentrums und unter Berücksichtigung der endlichen Positioniergenauigkeit des Implantates in der Hornhaut, an eine Position entlang jener Linie in der Hornhaut gebracht wird, welche das optische Zentrum, also den Verlauf der optischen Achse durch die Hornhaut repräsentiert.

Im Gegensatz zum Stand der Technik wird bewusst auf eine optische Abbildung durch das Implantat verzichtet. Die optische Wirkung des Implantates ist daher indirekt und durch den Verlauf des Übergangbereichs im angrenzenden Gewebe bestimmt. Da das erfindungsgemäße Implantat keine direkte optische Abbildungsfunktion hat, benötigt es auch keine ausgeführten optischen Flächen. Der Verlauf der Oberflächen des Implantates kann beliebig sein und ergibt sich nicht aus optischen Gesetzmäßigkeiten wie bei Implantaten mit direkter optischer Wirkung. Die Geometrie des Implantates wird ausschließlich auf Grund geometrischer Überlegungen hinsichtlich Art und Weise der Gewebsverdrängung in der Umgebung des Implantates bestimmt. Aus dem Gesagten ergibt sich auch, dass ein erfindungsgemäßes Implantat gemäß einer bevorzugten Ausführungsvariante der Erfindung nicht unbedingt transparent zu sein braucht, sondern auch undurchsichtig (opak) oder teiltransparent und auch in einer beliebigen Farbe ausgeführt werden kann, um seine erfindungsgemäße Funktion wahrzunehmen. Da unter bestimmten Umständen (Geometrien) störende Oberflächenphänomene an den transparenten Flächen (ähnlich wie bei optischen Implantaten) denkbar sind, aber die Geometrie eventuell ein günstiges Gewebeverdrängungsverhalten zeigt, kann durch die Wegnahme oder Herabsetzung der Transparenz diese Nebenwirkung beseitigt werden. Bei farbiger Ausgestaltung des Hornhautimplantats hat sich die Farbe schwarz als besonders vorteilhaft herausgestellt, da es sich dann vom dahinterliegenden Schwarz der Pupille nicht abhebt.

In jedem Fall trägt bei einem erfindungsgemäßen Implantat, selbst wenn es aus einem transparenten Material ausgeführt ist, der Anteil der Lichtstrahlen, die das Implantat nach der Implantation in die Hornhaut durchdringen, nichts zur Bildwahrnehmung bei, d.h. er erzeugt kein wahrnehmbares Bild auf der Netzhaut. Dies ist unter anderem durch die Dimensionen und Abmessungen des erfindungsgemäßen Implantates, durch deren Geometrie, durch ihre Oberflächenbeschaffenheit, durch ihr Material, durch ihre Farbgebung, durch optische Verluste an den Grenzflächen zum umgebenden Gewebe sowie durch biologische Wechselwirkung mit dem umgebenden Gewebe gegeben. Dies gilt ganz besonders, wenn sich das Implantat schon eine gewisse Zeit im Gewebe befindet. Im Besonderen ist die Wirkung des Implantates, im Gegensatz zum Stand der Technik, dadurch unempfindlich gegen optische und biologische Oberflächenphänomene.

Da das Auge den Gesetzen der geometrischen Optik folgt, und diese im Wesentlichen die Optik der achsennahen Strahlen ist, würde der Fachmann erwarten, dass durch das Einbringen eines nichtoptischen Körpers, der eigentlich ein optisches Hindernis darstellt, ins optische Zentrum der Hornhaut die Sehleistung erheblich beeinträchtigt werden würde. Es zeigte sich jedoch überraschender Weise, dass, wenn dieser Körper die erfindungsgemäßen Merkmale aufweist, diese Beeinträchtigung sich unmerklich auswirkt.

Bei der Erfindung ist das Verhältnis von Breite zu Wirkdicke eines erfindungsgemäßen Implantats kleiner als drei und größer als eins ist. Es hat sich gezeigt, dass in diesem Fall besonders gute Ergebnisse betreffend die Multifokalität erzielt werden können.

Wirkdicken von weniger als 500µm bzw. Breitenschwankungen von höchsten 30% der größten Breite tragen ebenfalls dazu bei, den Oberflächenverlauf der Hornhaut an die Anforderungen der multifokalen Abbildung anzupassen.

Bei der Erfindung ist das Hornhautimplantat rotationssymmetrisch um die Achse entlang der Wirkdicke ausgeführt, wobei eine besonders bevorzugte Variante der Erfindung vorsieht, dass die Hornhautimplantate kugelförmig ausgebildet sind, wodurch die Ausbildung des asphärischen Oberflächenverlaufs auf der Hornhautoberfläche ganz besonders gut gelingt.

Mit der vorliegenden Erfindung ist es möglich, ein Verfahren zur Korrektur der Fehlsichtigkeit des menschlichen Auges, insbesondere eine Korrektur der Altersichtigkeit alleine oder in Kombination mit Weitsichtigkeit durch Implantation eines Hornhautimplantates im optischen Zentrum des menschlichen Auges durchzuführen, ohne dass die Gefahr der Beeinträchtigung der Funktion des Implantates durch Einlagerungen im optischen Zentrum der Hornhaut um das Implantat besteht bzw. ohne dass aufwändige optische Linsen als Implantat zur Verfügung stehen müssen.

Dabei werden ein oder mehrere in Bezug auf das menschliche Auge keine Abbildungsfunktion besitzenden Hornhautimplantate mit je einer Wirkdicke, gemessen in Richtung der optischen Achse des Auges, von mehr als 50µm und je einer größten Breite, gemessen in einer Normalebene zur Dickenrichtung, von weniger als 0,5 mm, in das optische Zentrum der Hornhaut des menschlichen Auges eingebracht, so dass die Krümmung der Oberfläche der Hornhaut um das optische Zentrum der Hornhaut durch Auslenkung der Oberfläche der Hornhaut in deren optischem Zentrum verändert wird.

Durch das erfindungsgemäße Hornhautimplantat wird auch ein Verfahren zur Korrektur der Fehlsichtigkeit des menschlichen Auges, insbesondere eine Korrektur der Altersichtigkeit in Kombination mit Kurzsichtigkeit durch Implantation eines Hornhautimplantates im optischen Zentrum des menschlichen Auges ermöglicht, ohne dass die Gefahr der Beeinträchtigung der Funktion des Implantates durch Einlagerungen im optischen Zentrum der Hornhaut um das Implantat besteht bzw. ohne dass aufwändige optische Linsen als Implantat zur Verfügung stehen müssen.

Dabei werden eines oder mehrere in Bezug auf das menschliche Auge keine Abbildungsfunktion besitzende Hornhautimplantate mit einer Wirkdicke, gemessen in Richtung der optischen Achse des Auges, größer 50 µm und einer größten Breite, gemessen in einer Normalebene zur Dickenrichtung, kleiner 0,5 mm, im optischen Zentrum der Hornhaut in diese eingebracht, so dass die Oberfläche der Hornhaut in deren optischem Zentrum ausgelenkt wird, wobei zusätzlich außerhalb des optischen Zentrums der Hornhaut entweder mehrere Hornhautimplantate eingebracht werden oder ein ringförmiges Hornhautimplantat eingebracht wird, so dass die Krümmung der Hornhaut außerhalb deren optischen Zentrums verändert wird. Die erfindungsgemäßen Hornhautimplantate im optischen Zentrum der Hornhaut können dabei besonders vorteilhaft entsprechend den Ansprüchen 2, 3, 4 und/oder 5 ausgeführt sein.

Im Anschluss erfolgt nun eine detaillierte Beschreibung der Erfindung anhand von Ausführungsbeispielen, aus welchen weitere Vorteile der Erfindung für den Fachmann ersichtlich werden. Dabei zeigt:

### KURZE BESCHREIBUNG DER FIGUREN

- Fig.1: einen Querschnitt durch die Hornhaut eines menschlichen Auges mit implantiertem erfindungsgemäßen Hornhautimplantat
- Fig.2: einen Querschnitt durch die Hornhaut eines menschlichen Auges mit implantiertem erfindungsgemäßem Hornhautimplantat mit Kennzeichnung der unterschiedlich wirkenden Bereiche
- Fig.3: einen Querschnitt durch ein erfindungsgemäßes Implantat
- Fig.4: einen Querschnitt durch ein alternatives erfindungsgemäßes Hornhautimplantat
- Fig.5a-s: weitere alternative Querschnittsformen von Hornhautimplantaten
- Fig.6a-b: Kombination eines erfindungsgemäßen Hornhautimplantats mit einem ringförmigen Hornhautimplantat
- Fig.7a-b: Kombination eines erfindungsgemäßen Hornhautimplantats mit mehreren einzelnen Hornhautimplantaten
- Fig.8a-b: Kombination eines erfindungsgemäßen Hornhautimplantats mit mehreren einzelnen Hornhautimplantaten in Ausrichtung

### WEGE ZUR AUSFÜHRUNG DER ERFINDUNG

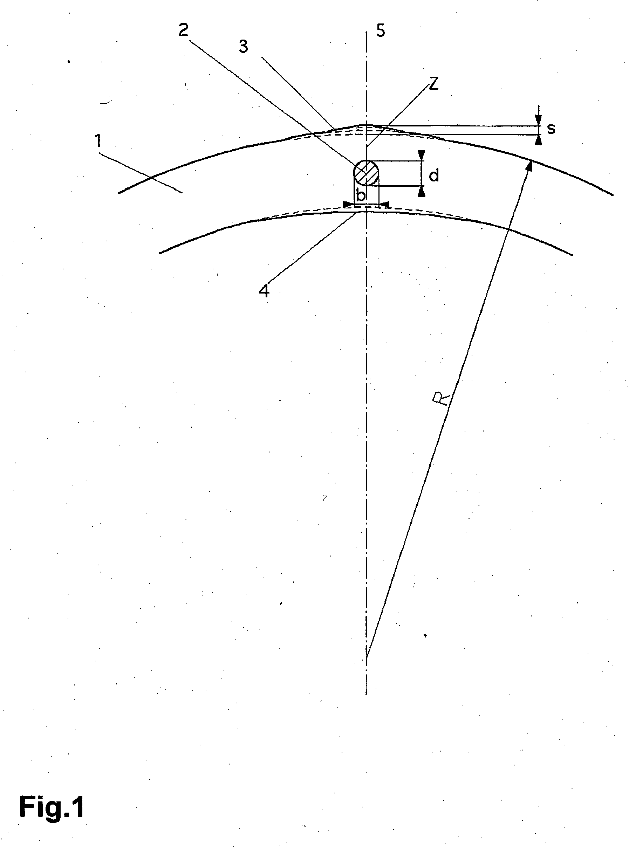

Fig.1 zeigt einen Querschnitt durch die, einen Krümmungsradius R aufweisende Hornhaut 1 eines menschlichen Auges samt optischem Zentrum Z. In das Hornhautgewebe der Hornhaut 1 ist ein erfindungsgemäßes Hornhautimplantat 2 implantiert, welches eine Wirkdicke d, gemessen in Richtung der optischen Achse 5 des Auges, größer als 50µm aufweist und eine Breite b, gemessen in einer Normalebene zur Dickenrichtung, kleiner als 0.5 mm aufweist.

Das Hornhautimplantat 2 besitzt in Bezug auf das menschliche Auge keine Abbildungsfunktion, d.h. einfallende Lichtstrahlen werden durch die optischen Eigenschaften des erfindungsgemäßen Hornhautimplantats 2 nicht auf der Netzhaut (nicht gezeichnet) des Auges abgebildet. Anstattdessen bewirkt die Implantation des Hornhautimplantats 2 eine zentrale Volumsaddition und dadurch einen asphärischen Oberflächenverlauf 3 der Hornhaut 1 um das optische Zentrum Z der Hornhaut, der auch eine multifokale Abbildung ermöglicht.

Im Gegensatz zu aus dem Stand der Technik bekannten Hornhautimplantaten und Verfahren zur Korrektur von Fehlsichtigkeiten wird erfindungsgemäß bewusst auf eine optische Abbildung durch das im optischen Zentrum Z des Auges implantierte Hornhautimplantat 2 verzichtet und die Korrektur der Fehlsichtigkeit ausschließlich durch Veränderung der Krümmung R der Hornhaut 1 um das Hornhautimplantat erzielt.

Dabei kommt es auch im Bereich der hinteren Hornhautfläche 4 zu Verformungen, die jedoch für die Korrektur der Fehlsichtigkeit von geringerer Bedeutung sind.

Erfindungsgemäß kann das Hornhautimplantat 2 von beliebiger Transparenz sein, d.h. es kann vollkommen undurchsichtig sein (opak), teiltransparent oder vollkommen transparent. Aufgrund der Tatsache, dass das Hornhautimplantat 2 in Bezug auf das Auge keine Abbildungsfunktion besitzt, kann es auch jede beliebige Farbe aufweisen, vorteilhafterweise schwarz, um gegenüber der schwarzen Pupille nicht störend zu wirken.

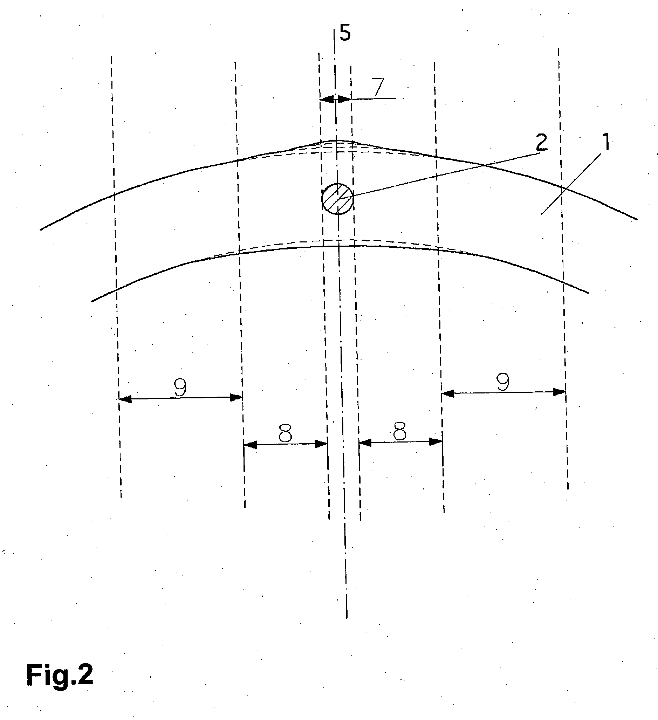

Fig.2 zeigt einen Querschnitt durch die Hornhaut 2 eines menschlichen Auges mit implantiertem erfindungsgemäßem Hornhautimplantat 2 samt Kennzeichnung der unterschiedlich wirkenden Bereiche der Hornhaut 1. Der zentrale Hornhautbereich 7, der durch die Abmessungen des Implantats 2 bestimmt ist, nimmt dabei nicht direkt am Sehakt teil. Der peripher anschließende Bereich 8 zeigt den asphärischen Oberflächenverlauf mit der daraus resultierenden multifokalen Abbildungseigenschaft. Daran grenzt der durch das Hornhautimplantat 2 unveränderte Hornhautbereich 9.

Je nach Abmessungen des Hornhautimplantats 2 kann im Bereich 8 eine Brechkraftaddition für das Nahsehen gegenüber dem Bereich 9 erzeugt werden. Der Bereich 9 ist für das Sehen in der Ferne geeignet. Er wird peripher durch den Pupillendurchmesser begrenzt.

Im Ausführungsbeispiel gemäß den Fig.1 und 2 wurde rein beispielhaft der Einsatz eines rotationssymmetrisch um die Achse der Wirkdicke geformten, nämlich kugelförmigen Hornhautimplantats 2 dargestellt, dessen Abmessungen somit mit einem Durchmesser von 0,5 mm begrenzt sind. Mit einem solchen kugelförmigen Hornhautimplantat 2 sind besonders gute Ergebnisse erzielbar, was den asphärischen Oberflächenverlauf 3 der Hornhaut 2 betrifft und damit die multifokalen Abbildungseigenschaften.

Für den Fachmann ist es jedoch klar, dass ein erfindungsgemäßes Hornhautimplantat 2 prinzipiell beliebige Gestalt aufweisen kann und dennoch die der Erfindung zugrunde liegende Aufgabe lösen kann, soferne nur das Implantat eine Wirkdicke, gemessen in Richtung der optischen Achse 5 des Auges, von 50µm nicht unterschreitet und eine Breite, gemessen in einer Normalebene zur Dickenrichtung von 0,5 mm nicht überschreitet.

Wichtig ist, dass aufgrund des im optischen Zentrum der Hornhaut 1 eingebrachten Hornhautimplantats 2 eine signifikante Änderung der Krümmung R der Hornhaut um das optische Zentrum derselben erfolgt.

Es ist vorgesehen, dass das Verhältnis zwischen Breite und Wirkdicke des Hornhautimplantats den Faktor 3 nicht überschreitet und den Faktor 1 nicht unterschreitet, um für den Patienten akzeptable multifokale Abbildungseigenschaften zu gewährleisten. Bevorzugte keine ist vorgesehen, dass die Breite entlang des Umfanges um nicht mehr als 30% der größten Breite schwanken darf.

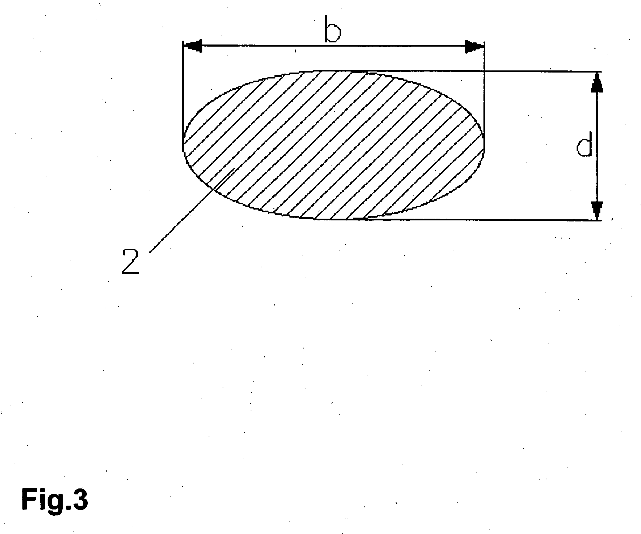

Fig.3 zeigt einen Querschnitt durch ein erfindungsgemäßes Hornhautimplantat 2 mit elliptischem Querschnitt mit Wirkdicke d und Breite b.

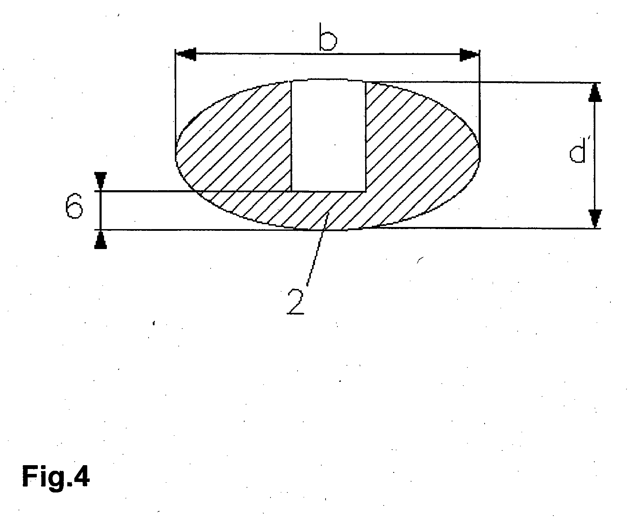

Fig.4 zeigt einen Querschnitt durch ein alternatives erfindungsgemäßes Hornhautimplantat 2, mit welchem derselbe erfindungsgemäße Effekt erzielbar ist, wie mit dem in Fig. 3 gezeigten Hornhautimplantat 2, solange nur die beschriebene minimale Wirkdicke von 50µm und maximale Breite von 0,5 mm eingehalten werden. Für den Fachmann ist es sofort ersichtlich, dass Hohlräume, wie beispielhaft in Fig. 4 schematisch eingezeichnet, zwar die Dicke des Hornhautimplantates 2 auf eine minimale Dicke 6 abschnittsweise verringern, die Wirkdicke d davon aber nicht beeinflusst wird daher kein Einfluss auf die Erzielung des erfindungsgemäßen Effektes ausgeübt wird, so dass auch mit einem Hornhautimplantat 2 wie in Fig.4 dargestellt eine Veränderung der Krümmung R der Hornhaut 1 im optischen Zentrum der Hornhaut 1 erzielbar ist ohne gleichzeitig die Sehfähigkeit zu beeinträchtigen.

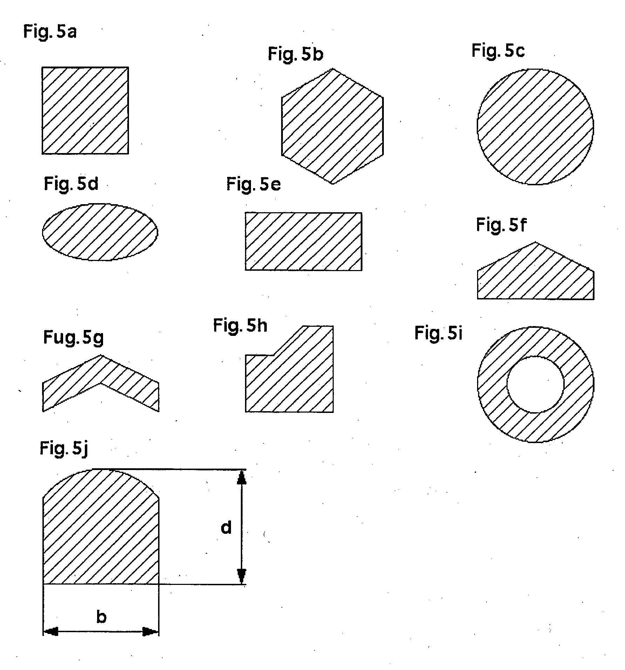

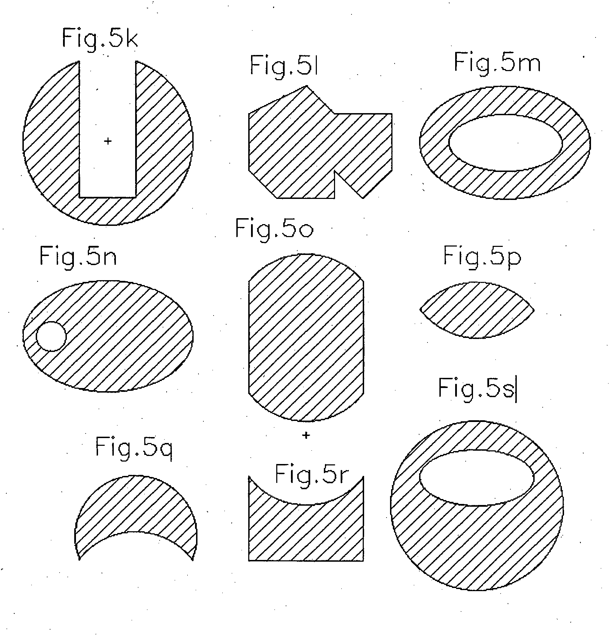

Fig.5a bis 5s zeigen beispielhaft weitere mögliche Querschnitte von Hornhautimplantaten 2, welche, sofern sie den Anforderungen an die mindesterforderliche Wirkdicke und der maximal erlaubten Breite genügen, den für das multifokale Sehen erforderlichen aspährischen Oberflächenverlauf 3 der Hornhaut 1 ermöglichen.

Die Erfindung beruht auf dem Umstand, dass eine Auslenkung der Hornhaut 1 erforderlich ist, um die gewünschte Wirkung zu erzielen, d.h. die Außenabmessungen des Hornhautimplantates 2, sowie dessen Elastizität sind so auf die Elastizität der Hornhaut 1 und auf die Kompression im Gewebe abzustimmen, dass die gewünschte Auslenkungen bzw. der damit verbundene asphärische Oberflächenverlauf 3 erzielt wird. Dies kann mit einem Hornhautimplantat mit einer Wirkdicke von mehr als 50µm erreicht werden, wobei durch Limitierung der Breite auf unter 0,5 mm eine relevante Beeinträchtigung der Sehfunktion sowie eine Ernährungsstörung der Hornhaut hintangehalten werden kann und eine Implantation im optischen Zentrum ermöglicht.

Als Material für das Implantat kann jeder biokompatible Stoff verwendet werden, beispielsweise PMMA, HEMA, Materialien auf Acrylbasis, Kunststoffe, Metalle, Halbleiter, Isolatoren oder andere an sich bekannte Materialien für dieses Einsatzgebiet.

Das Verfahren zur Erzeugung des Implantatbettes unterscheidet sich nicht von an sich bekannten Verfahren. Das Implantatbett kann beispielsweise durch ein LASIK Keratom, durch eine weitgehend abgeschlossene Hornhauttasche, wie beispielsweise in der EP 1 620 049 B1 beschrieben oder einen schmalen Hornhauttunnel hergestellt werden.

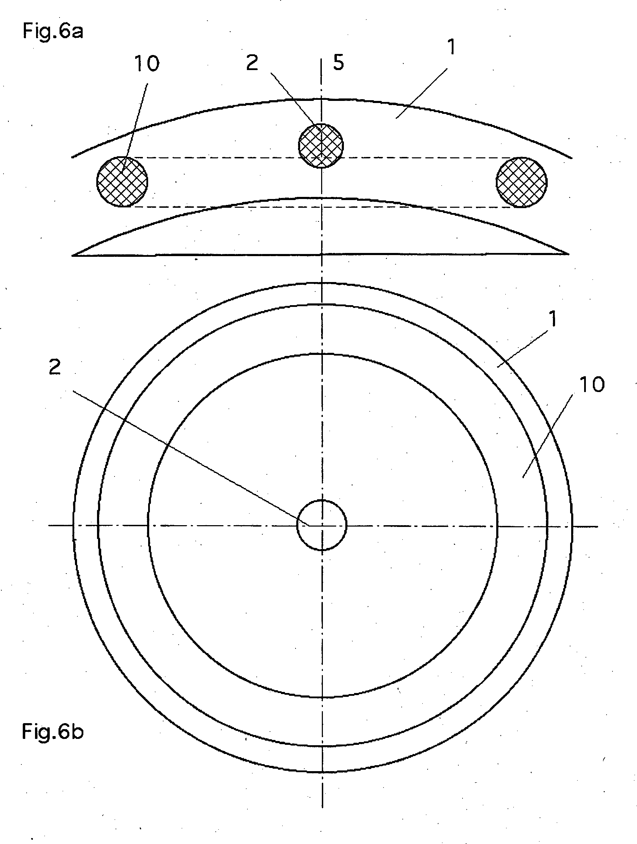

Das erfindungsgemäße Hornhautimplantat 2 dient primär zur Behebung von Alterssichtigkeit aber auch von Alterssichtigkeit in Kombination mit Weitsichtigkeit. Es kann aber auch in Kombination mit bereits bekannten anderen Hornhautimplantaten eingesetzt werden, beispielsweise in Kombination mit Ringimplantaten, wie dies in Fig.6 gezeigt ist, wodurch dann auch Alterssichtigkeit gemeinsam mit Kursichtigkeit korrigiert werden kann.

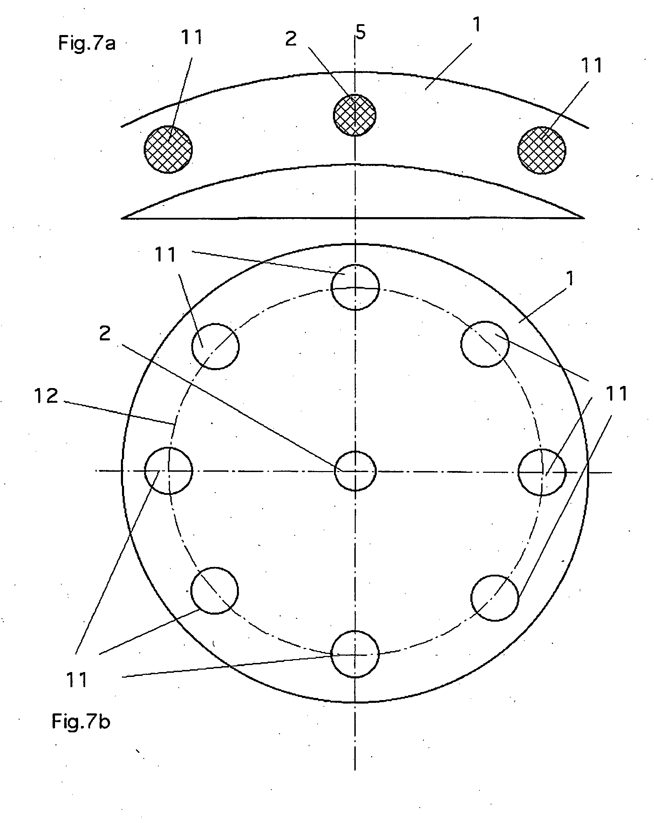

Fig.6a-b zeigen ein an sich bekanntes ringförmiges Hornhautimplantat 10, das außerhalb des optischen Zentrums der Hornhaut 1 implantiert ist, sowie ein erfindungsgemäßes Hornhautimplantat 2, welches im optischen Zentrum der Hornhaut 1 implantiert ist und den asphärischen Oberflächenverlauf 3 bewirkt. Anstelle eines ringförmigen Hornhautimplantats 10 können außerhalb des optischen Zentrums der Hornhaut 1, auch einzelne Kleinimplantate 11 wie in den Fig.7a-b gezeigt, zum Einsatz kommen.

Gemeinsam bewirken die Hornhautimplantate 2 und 10 bzw. 11 die Behebung von Alterssichtigkeit in Kombination mit Kurzsichtigkeit aufgrund der Änderung der Krümmung der Hornhautoberfläche, ohne dass die Implantate eine optische Wirkung aufweisen.

In Fig.7 sind die Kleinimplantate 11, entlang einer kreisförmig um das optische Zentrum verlaufende Linie 12 angeordnet. Haben die Kleinimplantate 11 unterschiedliche Dimensionen kann zusätzlich zur Alterssichtigkeit und Kurzsichtigkeit auch noch Astigmatismus korrigiert werden.

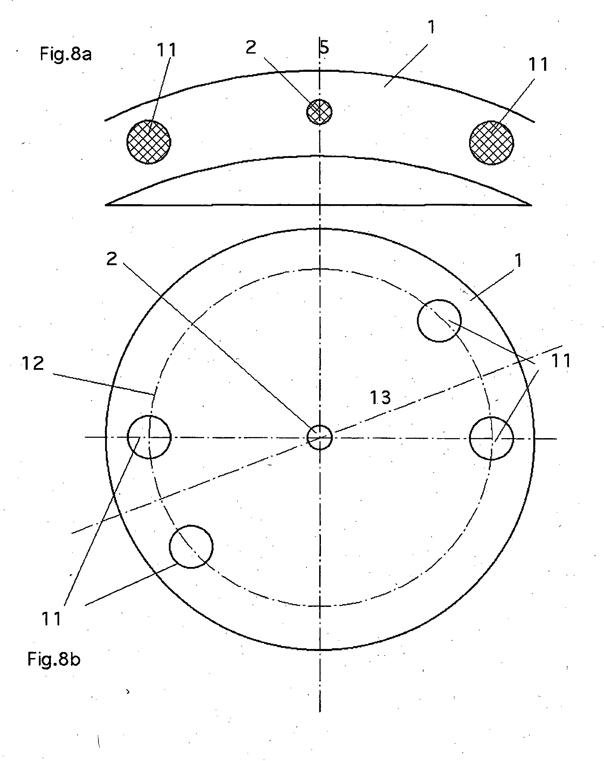

Ordnet man die Hornhautimplantate 2 und 11 wie in Fig.8 gezeigt an, so dass sich eine Vorzugsachse 13 ausbildet, so ist auch eine Astigmatismuskorrektur möglich.

Aufgrund der komplexen Krümmungsverläufe der Hornhautoberflächen in den Fig.6,7,8 wird die Oberfläche jeweils nur schematisch, ohne durch die Hornhautimplantate 2, 10 und 11 veränderte Krümmung gezeigt.

Im Besonderen wird festgehalten, dass Elemente von erfindungsgemäßen Ausführungsvarianten mit Elementen anderer erfindungsgemäßer Ausführungsvarianten kombiniert werden können, und diese Kombinationen wieder erfindungsgemäße Ausführungsvarianten darstellen.

## Patentansprüche

1. Hornhautimplantat zur Einbringung in das optische Zentrum (Z) der Hornhaut des menschlichen Auges zur Behebung von Fehlsichtigkeiten, insbesondere von Alterssichtigkeiten oder von Altersichtigkeiten in Kombination mit Weitsichtigkeiten oder Kurzsichtigkeiten, wobei
das Hornhautimplantat rotationssymmetrisch um die Achse entlang der Wirkdicke (d) ausgeführt ist, Wobei die Wirkdicke (d) als größte Dicke des Hornhautimplantats (2), gemessen in Richtung der Achse entlang der Wirkdicke mehr als 50 µm und weniger als 500 µm beträgt
und die größte Breite (b), gemessen in einer Normalebene zur Dickenrichtung, kleiner als 0,5 mm ist,
wobei das Verhältnis zwischen Breite (b) und Wirkdicke (d) des Hornhautimplantates den Faktor drei nicht überschreitet und den Faktor 1 nicht unterschreitet, und
wobei das Hornhautimplantat (2) in Bezug auf das menschliche Auge keine Abbildungsfunktion besitzt und wobei die Achse entlang der Wirkdicke (d) im implantierten Zustand des Hornhautimplantats in Richtung der optischen Achse (5) des Auges verläuft.

2. Hornhautimplantat nach Anspruch 1, **dadurch gekennzeichnet, dass** es die Form einer Kugel aufweist.

3. Hornhautimplantat nach Anspruch 1, **dadurch gekennzeichnet, dass** es einen elliptischen Querschnitt aufweist.

4. Hornhautimplantat nach einem der Ansprüche 1 bis 3, **dadurch gekennzeichnet, dass** es opak oder teiltransparent ist.

5. Hornhautimplantat nach einem der Ansprüche 1 bis 4, **dadurch gekennzeichnet, dass** es schwarz ist.

## Claims

1. A corneal implant for introduction into the visual centre (Z) of the cornea of the human eye for the purpose of rectifying impaired vision, in particular presbyopia, or presbyopia in combination with hypermetropia or myopia, wherein the corneal implant is arranged in a rotation-symmetrical manner about the axis along the effective thickness (d), wherein the effective thickness (d), as the largest thickness of the corneal implant (2) as measured in the direction of the axis along the effective thickness, is more than 50 µm and less than 500 µm, and the largest width (b), as measured in a normal plane in relation to the direction of the thickness, is less than 0.5 mm, wherein the ratio between width (b) and effective thickness (d) of the corneal implant does not exceed the factor of three and does not fall below the factor of one, and wherein the corneal implant (3) does not have any retinal imaging function, and wherein the axis along the effective thickness (d) extends in the implanted state of the corneal implant in the direction of the optical axis (5) of the eye.

2. A corneal implant according to claim 1, **characterized in that** it has the shape of a sphere.

3. A corneal implant according to claim 1, **characterized in that** it has an elliptical cross section.

4. A corneal implant according to one of the claims 1 to 3, **characterized in that** it is opaque or partially transparent.

5. A corneal implant according to one of the claims 1 to 4, **characterized in that** it is black.

## Revendications

1. Implant cornéen destiné à être inséré dans le centre optique (Z) de la cornée de l' oeil humain afin de corriger les défauts de vision, en particulier la presbytie ou la presbytie associée à l'hypermétropie ou à la myopie, dans lequel
l'implant cornéen a une forme symétrique en rotation autour de l'axe le long de l'épaisseur active (d),
l'épaisseur active (d) représentant la plus grande épaisseur de l'implant cornéen (2) mesurée dans le sens de l'axe le long de l'épaisseur active est de plus de 50 µm et moins de 500 µm,
et la plus grande largeur (b), mesurée dans un plan perpendiculaire au sens de l'épaisseur, étant inférieure à 0,5 mm,
dans lequel le rapport entre la largeur (b) et l'épaisseur active (d) de l'implant cornéen ne dépasse pas un facteur trois et n'est pas inférieur à un facteur un, et
dans lequel l'implant cornéen (2) ne possède pas de fonction d'image par rapport à l'oeil humain et l'axe le long de l'épaisseur active (d) est orienté en direction de l'axe optique (5) de l'oeil quand l'implant cornéen est implanté.

2. Implant cornéen selon la revendication 1, **caractérisé en ce qu'**il présente la forme d'une sphère.

3. Implant cornéen selon la revendication 1, **caractérisé en ce qu'**il présente une section elliptique.

4. Implant cornéen selon l'une des revendications 1 à 3, **caractérisé en ce qu'**il est opaque ou partiellement transparent.

5. Implant cornéen selon l'une des revendications 1 à 4, **caractérisé en ce qu'**il est noir.
